# EUROPEAN PATENT APPLICATION

(11) **EP 4 445 842 A1**
(43) Date of publication of application: **16.10.2024**
(21) Application number: 22903916.9
(22) Date of filing: 28.10.2022
(51) Int. Cl.: A61B 5/245

(54) **BIOMAGNETISM MEASUREMENT DEVICE**

(30) Priority: 10.12.2021 JP 2021200993
(71) Applicant: Shimadzu Corporation, Nakagyo-ku, Kyoto-shi, Kyoto 604-8511 (JP)
(72) Inventor: GOMI, Tomohiro, Kyoto-shi, Kyoto 604-8511 (JP); TOMITA, Sadamu, Kyoto-shi, Kyoto 604-8511 (JP); SHINADA, Kei, Kyoto-shi, Kyoto 604-8511 (JP)
(74) Representative: Kilian Kilian & Partner mbB
(86) International application number: PCT/JP2022/040419
(87) International publication number: WO 2023/105984

(57) **Abstract**

Provided is a biomagnetism measurement apparatus in which sufficient consideration is given to the fact that optimal arrangement of a sensor with respect to a subject varies depending on the purpose of the measurement. The biomagnetism measurement apparatus of the present invention has the following configuration. That is, the biomagnetism measurement apparatus of the present invention includes: a magnetic sensor group that is disposed outside a body of a subject and measures biomagnetism of the subject; and an arithmetic device 12 that acquires an arrangement pattern of the magnetic sensor group corresponding to a content of the measurement based on information indicating the content of the measurement.

## Description

### Technical Field

The present invention relates to a biomagnetism measurement apparatus.

### Background Art

A biomagnetism measurement apparatus capable of detecting weak magnetism emitted from a subject and, for example, visualizing a distribution of a magnetic field is expected to be able to perform measurement for various purposes. Such a biomagnetism measurement apparatus includes a plurality of sensors, and acquires a distribution of a magnetic field in a subject based on a signal output from each sensor.

### Citation List

### Patent Literature

### Patent Literature 1: JP 2021-148409 A

### Summary of Invention

### Technical Problem

However, in the conventional biomagnetism measurement apparatus, it is not sufficiently considered that optimal arrangement of the sensors with respect to the subject varies depending on the purpose of the measurement.

The present invention has been made in view of such circumstances, and an object of the present invention is to provide a biomagnetism measurement apparatus in which an arrangement of sensors is considered in accordance with the purpose of the measurement to be performed.

### Solution to Problem

In order to achieve such an object, the present invention has the following configuration. That is, the biomagnetism measurement apparatus comprising:
a group of magnetic sensors that are disposed outside a body of a subject and configured to measure biomagnetism of the subject; and
a processor configured to acquire, based on information indicating the content of the measurement, an arrangement pattern of the group of magnetic sensors such that the arrangement pattern corresponds to a content of the measurement to be performed.

### Advantageous Effects of Invention

A biomagnetism measurement apparatus of the present invention includes an arithmetic device that acquires an arrangement pattern of the magnetic sensor group corresponding to a content of the measurement based on information indicating the content of the measurement. In the biomagnetism measurement apparatus of the present invention, the arrangement of the sensors is sufficiently considered according to the purpose of the measurement.

### Brief Description of Drawings

FIG. 1 is a functional block diagram illustrating a configuration of a biomagnetism measurement apparatus according to an embodiment.
FIG. 2 is a schematic diagram illustrating an input of information indicating a content of measurement according to the embodiment.
FIG. 3 is a table referred to by an arrangement information output unit according to the embodiment.
FIG. 4 is a table referred to by the arrangement information output unit according to the embodiment.
FIG. 5 is a schematic diagram illustrating an output of an arrangement pattern according to the embodiment.
FIG. 6 is a schematic diagram illustrating an output of an arrangement pattern according to a modification.
FIG. 7 is a schematic diagram illustrating an example of an arrangement pattern.

### Description of Embodiments

Hereinafter, a biomagnetism measurement apparatus according to an embodiment will be described with reference to the drawings. A biomagnetism measurement apparatus 1 according to the embodiment includes a helmet 2 that covers the head of a subject and a plurality of sensors 3 that are inserted into any one of a plurality of through holes 2a provided in the helmet 2. The sensors 3 have such high sensitivity that biomagnetism can be detected, and can be attached to and detached from the helmet 2. Specifically, as the sensor 3, for example, an optical pumping module capable of sensing biomagnetism at room temperature can be applied. In addition, another configuration such as a diamond quantum sensor may be used for the sensor 3. The through holes 2a of the helmet 2 are numbered from the necessity of distinction. Similarly, the sensors 3 are numbered from the necessity of distinction.

### [Embodiment]

The biomagnetism measurement apparatus 1 includes an input unit 11 that causes a user to input information indicating the content of measurement and clinical information of a subject, an arithmetic device 12 that acquires an arrangement pattern of the sensor 3 corresponding to the information indicating the content of measurement and the clinical information of the subject, a storage unit 13 that stores a table to be referred to when the arithmetic device 12 operates, and a monitor 14 that displays the arrangement pattern. In addition, the biomagnetism measurement apparatus 1 includes an image generation unit 21 that images the output of each of the sensors 3. An image such as a magnetoencephalography generated by the image generation unit 21 is displayed on the monitor 14 described above.

The arithmetic device 12 and the image generation unit 21 are implemented by a central processing unit (CPU) executing various programs. Each unit may be realized by a single CPU or may be realized by a plurality of independent processors. In addition, the storage unit 13 stores not only the above-described table but also various types of information necessary for the operation of the biomagnetism measurement apparatus 1. Each of the units 12 and 21 operates by acquiring these pieces of information.

The biomagnetism measurement apparatus 1 is characterized in that measurement for various purposes can be performed. When biomagnetism is measured, since the arrangement of the sensors 3 suitable for measurement changes according to the purpose of the measurement, the biomagnetism measurement apparatus 1 according to the embodiment makes the positions of the sensors 3 variable so that the arrangement of the sensors 3 with respect to the subject can be changed. That is, the sensors 3 of the embodiment are inserted into the through holes 2a of the helmet 2 according to the purpose of the measurement. In this way, each sensor 3 can be appropriately arranged according to the purpose of the measurement.

In a case where the sensors 3 is simply attached to the helmet 2, there are many attaching methods of the sensors 3 to the helmet 2 conceivable. However, the image generation unit 21 at the time of executing a specific measurement operates on the assumption that each sensor 3 is disposed on the helmet 2 on the basis of, for example, only one attaching method among the plurality of methods. Since only one sensor 3 can be inserted into each through hole 2a, it is assumed that a sensor 3 having a number corresponding to the number of the through hole 2a is inserted into the through hole 2a in the case of performing specific measurement. In addition, the image generation unit 21 at the time of executing a measurement different from the specific measurement may operate on the assumption that each sensor 3 is disposed in each through hole 2a on the basis of an attaching method different from the specific measurement. The correspondence relationship between the number of the through hole 2a and the number of the sensor 3 in the attaching method at this time is not necessarily the same as the correspondence relationship at the time of performing the specific measurement described above.

In the configuration of the embodiment, when the information indicating the content of the measurement is acquired, the attaching method of each sensor 3 corresponding to the content of the measurement can be displayed on a display screen 14a of the monitor 14. Hereinafter, an operation when the biomagnetism measurement apparatus 1 displays an attaching method of the sensor 3 on the display screen 14a will be described.

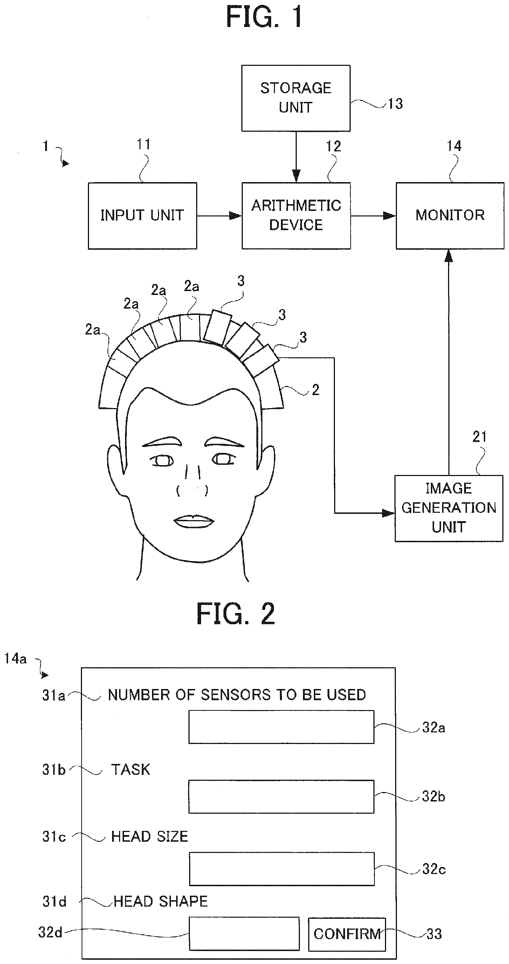

First, before attaching each sensor 3 to the helmet 2, a user inputs information indicating the content of the measurement to the biomagnetism measurement apparatus 1 through the input unit 11. At this point, the user inputs the information regarding the content of the measurement to the biomagnetism measurement apparatus 1 while referring to the display screen 14a of the monitor 14. As illustrated in FIG. 2, the display screen 14a at this time displays a setting screen including a section 31a of "number of sensors to be used", a section 3 1b of "task" which is the content of measurement, a section 31c of "head size" which is the clinical information of the subject, and a section 31d of "head shape" which is the clinical information of the subject. The number of sensors to be used, the task, the head size, and the head shape are examples of information indicating the content of measurement in the present embodiment that affects the attaching method of the sensor 3 to the helmet 2. An input box 32a on which the user can input specific information regarding the item is displayed around the section 31a of "number of sensors to be used" on the display screen 14a. Similarly, an input box 32b on which the user can input information regarding the task is displayed around the item 3 1b of "task" on the display screen 14a, and an input box 32c in which the user can input information regarding the head size is displayed around the item 31c of "head size" on the display screen 14a. Then, an input box 32d on which the user can input information regarding the head shape is displayed around the item 31d of "head shape" on the display screen 14a.

The task of the present embodiment will be described. When biomagnetism of a subject is measured using the biomagnetism measurement apparatus 1, a predetermined problem may be imposed on the subject. Specific examples of the task include a problem of opening and closing the eyes of the subject under measurement, a problem of appreciating a specific video, and a problem of listening to a specific sound. The task is a measurement type when a predetermined problem is imposed. Therefore, a corresponding problem is set in each of the tasks.

Hereinafter, information indicating the content of measurement will be described. The information regarding the number of sensors input by the user to the biomagnetism measurement apparatus 1 through the input unit 11 indicates the number of sensors used at the time of measurement, and the user can increase or decrease the number of sensors used for measurement according to the purpose of the measurement.

The information regarding the task input by the user to the biomagnetism measurement apparatus 1 through the input unit 11 indicates a task corresponding to a problem performed at the time of measurement, and the user can select the number of sensors to be used for measurement according to the purpose of the measurement.

The information regarding the head size and head shape input by the user to the biomagnetism measurement apparatus 1 through the input unit 11 indicates, for example, the type of helmet used for measurement.

As a specific configuration of the input boxes 32a, 32b, 32c, and 32d, the user may input various types of information through a keyboard attached to the input unit 11, or the user may select a target item from a pull-down menu displayed on the input boxes 32a, 32b, 32c, and 32d through a pointing device attached to the input unit 11.

A confirm button 33 displayed on the display screen 14a is used after the user completes the input of each item. When an operation is performed on the confirm button 33, the input operation of the user is completed, and the information indicating the content of the measurement input by the user is sent to the arithmetic device 12.

### <Operation of arithmetic device 12>

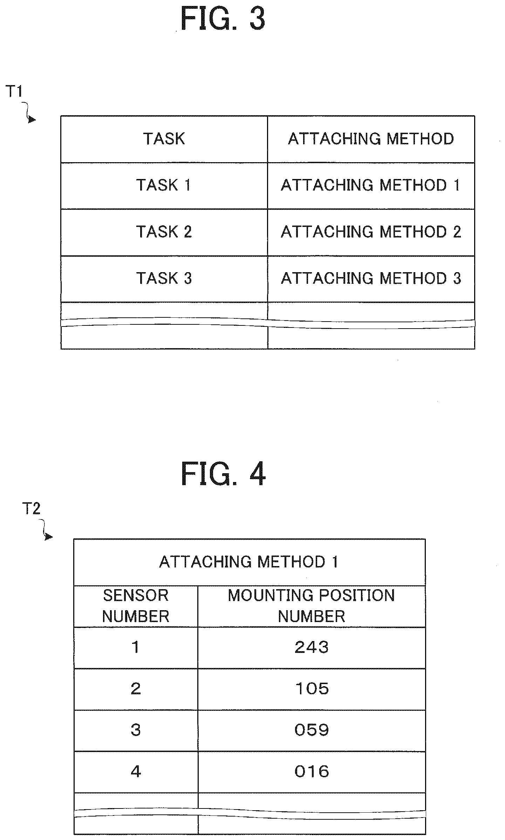

The arithmetic device 12 receives the information indicating the content of the measurement and outputs the information regarding the attaching method of the sensor 3 suitable for the measurement. FIG. 3 illustrates a table T1 referred to by the arithmetic device 12 at the time of operation. In the present embodiment, for convenience of description, it is assumed that the user can select only the task. The arithmetic device 12 outputs an attaching method of the sensor 3 corresponding to the task on the basis of the acquired information regarding the task. The attaching method corresponds to the arrangement pattern of the present invention.

In the table T1 referred to by the arithmetic device 12, various tasks and an optimal attaching method of the sensor 3 are associated with each other. For example, when the user selects the task 1 through the input unit 11, the arithmetic device 12 recognizes that an appropriate attaching method of the sensor 3 is an attaching method 1.

A table T2 illustrated in FIG. 4 is also referred to by the arithmetic device 12. The arithmetic device 12 that has recognized an appropriate sensor attaching method refers to the table T2 this time, and grasps a correspondence relationship between a sensor number specifically indicating the recognized attaching method and a mounting position number for identifying each of the through holes 2a of the helmet 2. In this example, since the attaching method 1 is an appropriate selection, the table of FIG. 4 illustrates a correspondence relationship related to the attaching method 1. However, in practice, the storage unit 13 stores a table similar to that in FIG. 4 for all the attaching methods illustrated in the table T1, and the arithmetic device 12 operates with reference to a table indicating an attaching method corresponding to a task selected by the user among the plurality of tables.

Note that, for the setting items other than the task described with reference to FIG. 2, similarly to the case of the task, the storage unit 13 stores a relevant table corresponding to the table T1. Therefore, the arithmetic device 12 of the present embodiment can grasp an appropriate attaching method for items other than the task by the same operation as in the case of the task. Since the table T2 is created for each attaching method and stored in the storage unit 13, the arithmetic device 12 can recognize the correspondence relationship between the appropriate sensor number and mounting position number by the operation similar to the case of the task when acquiring the attaching method. Note that a term relating to the type of the helmet 2 may be provided on the table T2, and the arithmetic device 12 may recognize an appropriate type of the helmet 2 in accordance with the setting of the "head size" and the "head shape" by the user.

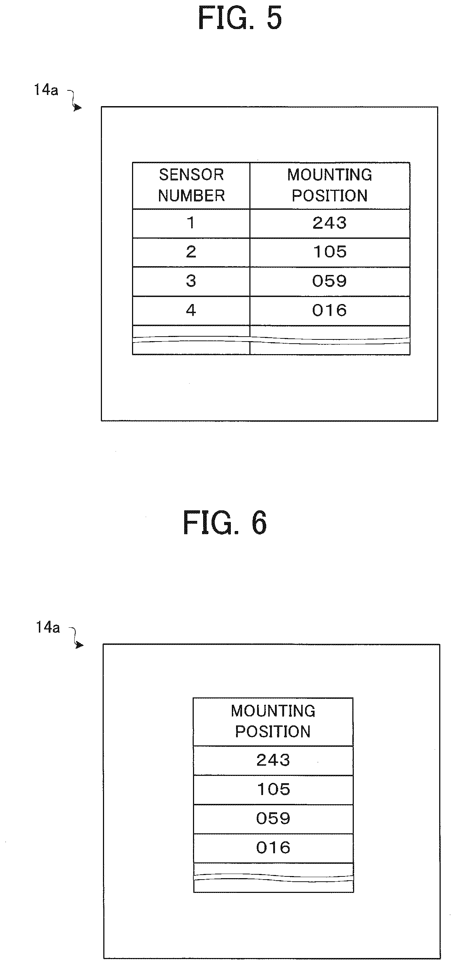

The arithmetic device 12 transmits the acquired information regarding the correspondence relationship to the monitor 14. As illustrated in FIG. 5, the monitor displays the contents of the table T2 on the display screen 14a. The user inserts each of the sensors 3 into the through hole 2a of the helmet 2 with reference to the display content. In this way, in the biomagnetism measurement apparatus 1 according to the embodiment, the arrangement of the sensors 3 used for measurement can be made appropriate according to the content of the measurement.

### <Effects of configuration of embodiment>

Hereinafter, a configuration and an effect of the biomagnetism measurement apparatus 1 in this example will be described.
(1) The biomagnetism measurement apparatus of this example includes: a magnetic sensor group that is disposed outside a body of a subject and measures biomagnetism of the subject; and an arithmetic device 12 that acquires an arrangement pattern of the magnetic sensor group corresponding to a content of the measurement based on information indicating the content of the measurement.
   As described above, by providing the arithmetic device 12 that acquires the arrangement pattern of the magnetic sensor group corresponding to the content of the measurement based on the information indicating the content of the measurement, the biomagnetism measurement apparatus 1 that can optimize the arrangement of the sensors according to the purpose of the measurement can be provided.
(2) The biomagnetism measurement apparatus according to (1), further includes a display unit that displays the arrangement pattern acquired by the arithmetic device 12.
   As described above, by providing the display unit that displays the arrangement pattern acquired by the arithmetic device 12, it is possible to provide the biomagnetism measurement apparatus 1 in which the user can easily grasp the arrangement pattern.
(3) In the biomagnetism measurement apparatus according to (1) or (2), the arithmetic device 12 acquires the arrangement pattern based on the information indicating a content of the measurement or clinical information of the subject.
   As described above, if the arithmetic device 12 acquires the arrangement pattern based on the information indicating the content of the measurement or the clinical information of the subject, it is possible to provide the biomagnetism measurement apparatus 1 that can instantaneously acquire the optimal arrangement of the sensors 3 that differs depending on the content of the measurement and the clinical information of the subject.
(4) The biomagnetism measurement apparatus according to any one of (1) to (3) further includes an input unit 11 that receives the information indicating a content of the measurement from a user.
   As described above, if the input unit 11 that receives the information indicating the content of the measurement from the user is provided, the user can instantaneously acquire the arrangement of the sensors 3 that matches the content of the measurement.
(5) The biomagnetism measurement apparatus according to (1) to (4) further includes a helmet 2 that holds the magnetic sensor 3 and is mounted on the subject, in which the information regarding the arrangement pattern is information regarding an attaching method when the sensor 3 is attached to the helmet 2.
   As described above, if the information regarding the arrangement pattern is information regarding an attaching method when the sensor 3 is attached to the helmet 2 mounted on the subject with the sensor 3 disposed, the user can instantaneously grasp the attaching method of the sensor 3 to the helmet 2.
(6) In the biomagnetism measurement apparatus according to (5), the information regarding the arrangement pattern includes information regarding a correspondence relationship between a specific sensor 3 and a specific mounting position on the helmet 2.
   As described above, if the information regarding the arrangement pattern includes the information regarding the correspondence relationship between the specific sensor 3 and the specific mounting position on the helmet 2, the user can instantaneously understand the correspondence relationship between the sensor number and the mounting position number.
(7) The biomagnetism measurement apparatus according to any one of (1) to (6) further includes a drive unit that receives the arrangement pattern acquired by the arithmetic device 12 and moves the sensor 3. Details of this example will be described later.
   As described above, by providing the drive unit that receives the arrangement pattern output from the arithmetic device 12 and moves the sensor 3, the arrangement of the sensor 3 is optimized according to the purpose of the measurement even if the user does not manually move the sensor 3.
(8) The biomagnetism measurement apparatus according to any one of (1) to (7) further includes a storage unit 13 that stores a table in which the information indicating the content of the measurement and the arrangement pattern are associated with each other.

As described above, by providing the storage unit 13 that stores the table in which the information indicating the content of the measurement and the arrangement pattern are associated with each other, the operation of the arithmetic device 12 becomes more reliable.

### <Other embodiments>

Note that the embodiment disclosed herein is illustrative in all respects and is not restrictive. The scope of the present invention includes the claims and all modifications within the meaning and scope equivalent to the claims. As an example, the present invention can be modified as follows.

(1) In the above-described embodiment, the sensor 3 is mounted on the helmet 2 manually by the user, but this may be automatic. The biomagnetism measurement apparatus 1 having such a configuration includes a mechanism for inserting the sensor 3 into the through hole 2a of the helmet 2. That is, according to the present modification, the drive unit that drives the sensor 3 is provided, and the drive unit receives the information regarding the attaching method of the sensor 3 output from the arithmetic device 12 and moves the sensor 3. In the present modification, it is not always necessary to display the information regarding the attaching method of the sensor 3 on the display screen 14a of the monitor 14.
(2) In the above-described embodiment, the correspondence relationship between the sensor number and the mounting position number is displayed on the display screen 14a of the monitor 14, but the present invention is not limited to this configuration. The present invention can also be applied to a configuration in which it is not necessary to distinguish the sensors 3. When each sensor 3 of the present modification is inserted into the through hole 2a in the helmet 2, the sensor 3 can acquire the mounting position number of the through hole 2a into which each sensor 3 is inserted based on an identification unit provided in the through hole 2a. Each sensor 3 grasps its own position in the helmet 2 according to the acquired mounting position number, and outputs information regarding the mounting position number to the image generation unit 21. Each sensor 3 is assigned a sensor number so that the image generation unit 21 can distinguish the sensor 3, and each sensor 3 transmits its own sensor number together with the mounting position number to the image generation unit 21. As a result, the image generation unit 21 can operate while grasping the correspondence relationship between the sensor number and the mounting position number. As the identification unit provided in the through hole 2a, a tag (for example, a barcode) or the like can be applied. In this example, the sensor 3 includes a scanner that reads a barcode, and operates by reading the barcode provided on the inner wall of the through hole 2a.
   In this example, since it is not necessary to distinguish the sensors 3 and mount the sensors 3 on the helmet 2, the content of the table displayed on the display screen 14a of the monitor 14 is only the information regarding the mounting position as illustrated in FIG. 6. Therefore, the table stored in the storage unit 13 is similar to this, and the terms of the sensor numbers of the table T2 illustrated in FIG. 4 are omitted.
(3) In the setting screen of the content of the measurement described in FIG. 2 of the above-described embodiment, for example, a task may be omitted. That is, the present invention can also be applied to a device that measures biomagnetism in a stable state in which no problem is imposed on a subject. The same applies to the other terms "number of sensors to be used", "head size", and "head shape" described in FIG. 2. In addition, as the content of the measurement, for example, a setting item not illustrated in FIG. 2 such as "disease state" may be added to the content of the measurement.
(4) In the above-described embodiment, the contents of the table T2 stored in the storage unit 13 are displayed on the display screen 14a of the monitor 14, but the present invention is not limited to this configuration. The arithmetic device 12 may edit the table T2 on the basis of information such as "head size" and" head shape", and transmit the edited table to the monitor 14. With this configuration, it is not necessary to prepare a table in advance for all conceivable cases, and a biomagnetism measurement apparatus having excellent flexibility can be provided.
(5) In the above-described embodiment, the contents of the table T2 stored in the storage unit 13 are displayed on the display screen 14a of the monitor 14, but the present invention is not limited to this configuration. The shape of the helmet 2 and the positions of the through holes 2a in the helmet 2 may be stored in the storage unit 13, and the arithmetic device 12 may generate a table on the basis of information such as "head size" and" head shape". With this configuration, it is not necessary to prepare a table in advance for all conceivable cases, and a biomagnetism measurement apparatus having excellent flexibility can be provided.

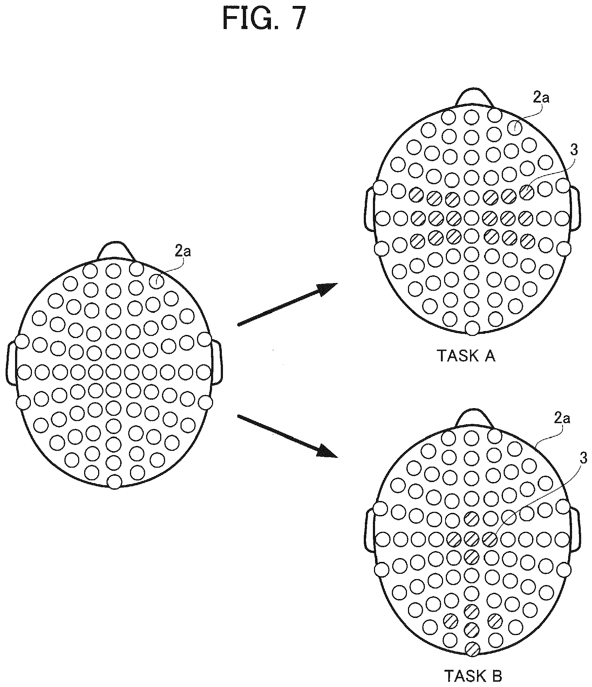

(6) In FIG. 1, the sensor 3 is not mounted on all of the through holes 2a. This indicates a mounting status of the sensor 3 when a predetermined task or the like is executed, and it is also conceivable that the sensor 3 is mounted on all of the through holes 2a for measurement depending on the task or the like. In addition, even in a case where the sensor 3 is not mounted on all the through holes 2a, the position of the through hole 2a into which the sensor 3 is not inserted may not be as illustrated in FIG. 1 depending on a task or the like. In addition, the number of through holes 2a, the number of sensors 3, and the like in the helmet 2 are all examples. FIG. 7 illustrates positions of the through holes 2a and the sensors 3 when the top of the subject is looked down, and illustrates arrangement of the sensors 3 in a task A and arrangement of the sensors in a task B. That is, in the task A, the sensors 3 are arranged in a 3 × 3 two-dimensional matrix on the left side and the right side of the helmet 2. On the other hand, in the task B, the sensors 3 are disposed at the top of the helmet 2 and front, rear, left, and right positions surrounding the top, and the sensors 3 are disposed at the rear of the helmet 2 and front, rear, left, and right positions surrounding the rear.
(7) Although the above-described embodiment has a configuration related to a magnetoencephalography, the present invention can also be applied to an apparatus capable of generating an image other than a magnetoencephalography, such as a magnetocardiogram.

### Reference Signs List

- 3: sensor (magnetic sensor)
- 11: input unit
- 12: arrangement information output unit

## Claims

1. A biomagnetism measurement apparatus comprising:
a group of magnetic sensors that are disposed outside a body of a subject and configured to measure biomagnetism of the subject; and
a processor configured to acquire, based on information indicating the content of the measurement, an arrangement pattern of the group of magnetic sensors such that the arrangement pattern corresponds to a content of the measurement to be performed.

2. The biomagnetism measurement apparatus according to claim 1, further comprising a display unit that displays the arrangement pattern acquired by the arithmetic device.

3. The biomagnetism measurement apparatus according to claim 1, wherein the arithmetic device acquires the arrangement pattern based on the information indicating a content of the measurement or clinical information of the subject.

4. The biomagnetism measurement apparatus according to claim 1, further comprising an input unit that receives the information indicating the content of the measurement from a user.

5. The biomagnetism measurement apparatus according to claim 1, further comprising a support that holds the magnetic sensor and is mounted on the subject,
wherein the information regarding the arrangement pattern is information regarding an attaching method when the magnetic sensor is attached to the support.

6. The biomagnetism measurement apparatus according to claim 5, wherein the information regarding the arrangement pattern includes information regarding a correspondence relationship between a specific magnetic sensor and a specific mounting position on the support.

7. The biomagnetism measurement apparatus according to claim 1, further comprising a drive unit that receives the arrangement pattern acquired by the arithmetic device and moves the magnetic sensor.

8. The biomagnetism measurement apparatus according to claim 1, further comprising a storage unit that stores a table in which the information indicating the content of the measurement and the arrangement pattern are associated with each other.
